(19) Europäisches Patentamt
European Patent Office
Office européen des brevets

(11) **EP 4 018 826 A1**

(12) **EUROPEAN PATENT APPLICATION**
published in accordance with Art. 153(4) EPC

(43) Date of publication:
**29.06.2022 Bulletin 2022/26**

(21) Application number: **20856450.0**

(22) Date of filing: **05.08.2020**

(51) International Patent Classification (IPC):
**A01N 1/02** (2006.01)  **C12M 1/00** (2006.01)
**C12N 5/071** (2010.01)

(52) Cooperative Patent Classification (CPC):
**A01N 1/02; C12M 1/00**

(86) International application number:
**PCT/JP2020/030049**

(87) International publication number:
**WO 2021/039330 (04.03.2021 Gazette 2021/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.08.2019 JP 2019152906**

(71) Applicant: **Mitsubishi Paper Mills Limited
Tokyo 1300026 (JP)**

(72) Inventors:
• **MATSUZAWA, Atsushi
Tokyo 130-0026 (JP)**
• **YOSHIDA, Kakeru
Tokyo 130-0026 (JP)**

(74) Representative: **Cabinet Beau de Loménie
158, rue de l'Université
75340 Paris Cedex 07 (FR)**

(54) **JIG FOR CELL OR TISSUE CRYOPRESERVATION**

(57) The present invention aims to provide a device for cryopreservation providing excellent working efficiency in operations for freezing and thawing cells or tissues. Provided is a device for cryopreservation of a cell or tissue including at least: a body including a strip-shaped tip; and a cap covering the tip of the body, the tip including at least a deposition part comprising a preservation solution absorber, the tip having a length in a minor axis direction of not shorter than 70% of an inner diameter of the cap, the cap including an inner cavity having a length in a major axis direction of not longer than 200% of a length in a major axis direction of the tip.

FIG.5

**Description**

TECHNICAL FIELD

The present invention relates to a device for cryopreservation of a cell or tissue.

BACKGROUND ART

Excellent cell or tissue preservation techniques are desired in various industrial fields. For example, in the bovine embryo transfer technology, embryos are cryopreserved in advance and thawed and transferred according to the estrous cycle of a recipient cow. In the human fertility treatment, eggs or ovaries harvested from a woman's body are cryopreserved until appropriate timing for transplantation, and the cryopreserved eggs or ovaries are thawed for use in transplantation.

Generally, cells or tissues harvested from living bodies gradually become inactive and/or undergo transformation even in a culture medium. Thus, long-term culture of cells or tissues in vitro is undesirable. For this reason, techniques for long-term preservation of cells or tissues with their bioactivity maintained are essential. Excellent preservation techniques enable more accurate analysis of cells or tissues harvested. Such excellent preservation techniques also enable transplantation of cells or tissues with their bioactivity maintained at a higher level, thus likely improving the engraftment rate. The techniques also enable sequential production and preservation of artificial tissues for transplantation, such as skins cultured in vitro and what is called "cell sheets" formed in vitro, and storage thereof until needed. Therefore, such excellent preservation techniques are expected to bring great advantages not only in the medical science fields but also in the industrial fields.

One of known cell or tissue cryopreservation methods is a slow freezing method, for example. In this method, cells or tissues are immersed in a preservation solution prepared by adding a cryoprotectant to a physiological solution such as phosphate buffered saline, for example. Examples of the cryoprotectant include compounds such as glycerol, ethylene glycol, and dimethyl sulfoxide. The cells or tissues immersed in the preservation solution are cooled down to -30°C to -35°C at a relatively slow cooling rate (for example, 0.3°C to 0.5°C/min), whereby the solution inside and outside the cells or tissues is sufficiently cooled and increases its viscosity. Further cooling down the cells or tissues in the preservation solution in such a state to the temperature of liquid nitrogen (-196°C) allows a slight amount of the solution both inside and outside (surrounding) the cells or tissues to become a solid while the amorphous state thereof is maintained, that is, to vitrify. Vitrification solidifies the solution inside and outside the cells or tissues, which substantially immobilizes the molecules. Thus, the vitrified cells or tissues can be semipermanently preserved in the liquid nitrogen.

Another known cell or tissue cryopreservation method is a vitrification method. The vitrification method is a technique using a principle that addition of a large amount of a cryoprotectant to a preservation solution lowers the freezing point of the preservation solution, which reduces or prevents ice crystal formation at sub-zero temperatures. When quickly cooled in liquid nitrogen, the preservation solution can solidify without forming ice crystals. This solidification is called vitrification. The preservation solution containing a large amount of a cryoprotectant is called a vitrification solution.

The slow freezing method described above requires cooling at a relatively slow cooling rate, which prolongs the operation of cryopreservation. Disadvantageously, this method also requires a device or jig for controlling the cooling rate. In addition, the slow freezing method cannot avoid ice crystal formation in the preservation solution outside the cells or tissues, which may cause physical damage to the cells or tissues. In contrast, the vitrification method described above is a process that shortens the operation time and requires no special devices or jigs. In addition, the vitrification method provides high viability because it prevents ice crystal formation.

There have been reported various examples of cell or tissue cryopreservation by the vitrification method using various methods and various cells or tissues. For example, according to Patent Literature 1, application of the vitrification method to animal or human reproductive or somatic cells is very useful in terms of viability after cryopreservation and thawing.

The vitrification method is a technique which has been developed mainly using human reproductive cells. More recently, its application to iPS or ES cells has also been widely examined. Non-Patent Literature 1 discloses the effectiveness of the vitrification method in preservation of Drosophila embryos. Patent Literature 2 discloses the effectiveness of the vitrification method in preservation of plant culture cells and tissues. As mentioned here, the vitrification method is known to be useful for preservation of various kinds of cells and tissues.

It is known that a higher freezing rate is better for suitable vitrification. Also in a thawing step after cryopreservation, it is known that a higher freezing rate is better for reduction or prevention of ice crystal reformation in cells or tissues.

The freezing rate and the thawing rate are important factors for suitable vitrification. Of these, the thawing rate is considered to be particularly important. For example, as described in Non-Patent Literature 2, it is known that rapidly frozen cells may have a low viability, if the thawing rate is slow. According to Patent Literature 3, the viability of human induced pluripotent stem (iPS) cell-derived neurons/precursor cells after thawing is improved by thawing frozen samples by the rapid thawing method.

As a general cryopreservation jig or freezing method applicable to the vitrification method, Patent Literature 4 suggests a method in which mammalian embryos or eggs are attached to an inner surface of a cryopreservation container such as a cryostraw, cryovial, or cryotube, with a vitrification solution in a minimum amount sufficient to cover these embryos or eggs, and the container is brought into contact with liquid nitrogen for rapid cooling. In a thawing method that is performed after the cryopreservation method, the cryopreservation container stored by the above-described method is taken out from the liquid nitrogen, and one end of the container is opened to inject a diluent at 33°C to 39°C into the container, whereby the frozen embryos or eggs are thawed and diluted. This cryopreservation jig or cryopreservation method is considered to be capable of preserving, thawing, and diluting mammalian embryos or eggs with high viability, without risk of infection with viruses and/or bacteria. However, it is highly difficult to attach embryos or eggs to an inner surface of a cryopreservation container such as a cryostraw, cryovial, or cryotube (freezing step), and it is also difficult to confirm that the embryos or eggs are deposited in the cryopreservation container. In the thawing step in which a diluent is used for thawing, it is also difficult to perform thawing while visually checking the position of the embryos or eggs deposited, leading to difficulty in secure recovery of the embryos or eggs. Moreover, the method requires special devices such as a straw sealer in the freezing step and a straw cutter in the thawing step, to complicate the process.

Patent Literature 5 discloses a cell cryopreservation tool including a cell holding member having a thermal conductive member and a tubular storage member. With this tool, the problems in Patent Literature 4 are solved to some degree. The cryopreservation tool disclosed in Patent Literature 5 is used in the following manner: eggs are attached to the cell holding member under a microscope; the cell holding member is housed in the tubular storage member; and the cryopreservation tool is then immersed in liquid nitrogen for vitrification. Subsequently, a lid is attached to an opening of the tubular storage member, and the tubular storage member is stored in a liquid nitrogen tank.

Patent Literature 6 and Patent Literature 7 each disclose a cryopreservation jig and a freezing and thawing method applicable to a vitrification method with fewer steps, which is called the Cryotop® method that has been used in the field of human fertility treatment. Freezing in these methods is performed using an egg cryopreservation tool including a flexible, clear, and colorless film strip as an egg-holding strip. Eggs or embryos are deposited with a very small amount of a preservation solution on the film under microscope observation, and the film with the eggs attached thereto is immersed in liquid nitrogen to be frozen. Upon thawing, eggs or embryos are thawed by immersing the egg-holding strip in a thawing solution kept warm, and the eggs or embryos deposited on the strip are recovered in the thawing solution. In this method, the eggs or embryos are deposited on the strip with a small amount of a preservation solution by operator's manual operation. It is known that this technique enables freezing of eggs or embryos with high viability though it involves highly difficult operation.

Patent Literatures 8 to 10 each suggest a method of cryopreserving cells or tissues with high viability, including depositing cells or tissues on a preservation solution remover with a preservation solution containing a large amount of a cryoprotectant, and removing an excess preservation solution surrounding the cells or tissues. Patent Literature 8 discloses a preservation solution absorber having a specific haze. Patent Literature 9 and Patent Literature 10 each disclose a vitrification cryopreservation jig including, as a preservation solution absorber, a porous sintered body or a porous structured body formed of a material having a specific refractive index.

Patent Literature 11 discloses a living cell cryopreservation tool which can omit an operation of removing an excess preservation solution owing to the configuration including: a strip-shaped light-transmitting base unit; and a defect part surrounded by a water absorbing unit (preservation solution absorber) at a portion of the base unit. According to a cryopreservation method of Patent Literature 11, eggs are deposited on the defect part with a small amount of a vitrification solution, and the cryopreservation jig including an egg-holding strip is entirely housed in a tubular storage container, and then immersed in liquid nitrogen for vitrification.

CITATION LIST

- Patent Literature

Patent Literature 1: JP 3044323 B
Patent Literature 2: JP 2008-5846 A
Patent Literature 3: JP 2017-104061 A
Patent Literature 4: JP 2000-189155 A
Patent Literature 5: JP 5798633 B
Patent Literature 6: JP 2002-315573 A
Patent Literature 7: JP 2006-271395 A

Patent Literature 8: JP 2014-183757 A
Patent Literature 9: JP 2015-142523 A
Patent Literature 10: WO 2015/064380
Patent Literature 11: WO 2019/004300

- Non-Patent Literature

Non-Patent Literature 1: Steponkus et al., Nature 345:170-172 (1990)
Non-Patent Literature 2: "Seisaibo no toketsu ni yoru shogai to hogo no kiko" (Mechanism of damage to living cells by freezing and protection) written by Hiroshi Souzu, Kagaku to Seibutsu (Chemistry and Biology), vol. 18 (1980), no. 2. pp. 78-87, published by The Japan Society for Bioscience, Biotechnology, and Agrochemistry

SUMMARY OF INVENTION

- Technical Problem

Patent Literature 5 discloses a cell cryopreservation tool including a cell holding member having a thermal conductive member and a tubular storage member. The process using this tool disadvantageously includes complicated operations such as attachment of a lid to the tubular storage member in the freezing step. The thawing step is also complicated in which the cell holding member housed in the tubular storage member is taken out, as it includes operations such as detachment of the lid and also requires an extraction tool.

There have been suggested cryopreservation jigs of cryopreserving eggs or embryos with a small amount of a preservation solution to provide high viability, for example, by limiting the width of the film on which eggs or embryos are to be deposited and vitrification methods with fewer steps (Patent Literatures 6 and 7). However, these methods have some disadvantages. In the freezing operation, eggs or embryos are dropped with a preservation solution onto the width-limited film, frozen, and then stored in a tubular storage member. Upon insertion of the end of the width-limited film into the tubular storage member, the solidified vitrification solution and the solidified eggs or embryos may contact the end face of the tubular storage member to unintendedly fall. Also, air bubbles are attached to the film during thawing in which the eggs or embryos are thawed and recovered, making it difficult to distinguish the deposited embryos or eggs from the air bubbles under microscope observation. Further, the air bubbles attached to the film may be attached to the eggs or embryos during the thawing operation, impairing their visibility, or the eggs or embryos may suddenly disappear from the microscopic field to be lost due to buoyancy of the air bubbles. These problems interfere with the recovery of the embryos or eggs.

Patent Literatures 8 to 10 each suggest a method of cryopreserving eggs or embryos with high viability by using a cryopreservation jig with absorbency to remove an excess preservation solution surrounding these reproductive cells. However, as is the case for Patent Literature 6 or Patent Literature 7, the problem concerning an influence of air bubbles generated during the thawing operation has not been solved.

Patent Literature 11 discloses a cryopreservation jig housing a body on which eggs are deposited in a tubular storage container. As is the case for Patent Literature 5, a cutting tool and/or an extraction tool may be required in the thawing step, which complicates an operation of taking out the housed cryopreservation jig. Consequently, the thawing step is complicated. Moreover, the problem of the possibility of falling of eggs or embryos deposited with a preservation solution upon housing into the tubular member remains unsolved as in Patent Literature 6 and Patent Literature 7.

The present invention mainly aims to provide a device for cryopreservation of a cell or tissue enabling cryopreservation of cells or tissues simply and securely. More specifically, the present invention aims to provide a device for cryopreservation of a cell or tissue that enables cryopreservation of eggs or embryos with a small amount of a preservation solution in the freezing step, while exhibiting excellent insertability which avoids a risk of falling of cells or tissues upon housing of a deposition part into a tubular member, requires no complicated operations in the thawing step which shortens the time for the thawing step, and reduce or prevent attachment of air bubbles thereto to provide excellent visibility during recovery of cells or tissues in a thawing solution.

- Solution to problem

As a result of extensive studies to solve the above problems, the present inventors found that a device for cryopreservation of a cell or tissue configured as described below (hereinafter, also referred to as "the device for cryopreservation of the present invention") can solve the above problems.

The present invention relates to a device for cryopreservation of a cell or tissue including at least: a body including a strip-shaped tip; and a cap covering the tip of the body, the tip including at least a deposition part including a preservation solution absorber, the tip having a length in a minor axis direction of not shorter than 70% of an inner diameter of the cap, the cap including an inner cavity having a length in a major axis direction of not longer than 200% of a length in a major axis direction of the tip.

- Advantageous Effects of Invention

The present invention provides a device for cryopreservation of a cell or tissue that enables cryopreservation of eggs or embryos with a small amount of a preservation solution in the freezing step, while exhibiting excellent insertability which avoids a risk of falling of eggs or embryos upon housing of the deposition part into the cap, requires no complicated operations in the thawing step which shortens the time for the thawing step, and provides excellent visibility during recovery of cells or tissues in the thawing solution.

BRIEF DESCRIPTION OF DRAWINGS

Fig. 1 is a schematic top view of an example of a body of a device for cryopreservation of the present invention.
Fig. 2 is a schematic side view of an example of the body of the device for cryopreservation of the present invention.
Fig. 3 is a side cross-sectional view of an example of a cap of the device for cryopreservation of the present invention.
Fig. 4 is a side view of an example of the cap of the device for cryopreservation of the present invention, as viewed from an opening side.
Fig. 5 is a schematic view of an example of the body and the cap being fitted together and fixed.
Fig. 6 is a schematic side view of a cell and a preservation solution being deposited on the device for cryopreservation of the present invention.
Fig. 7 is a schematic side view of the cap being fitted and fixed after the cell and the preservation solution are deposited on the device for cryopreservation of the present invention.
Fig. 8 is a schematic cross-sectional view of an example of a fixture used in a freezing and thawing method that uses the device for cryopreservation of the present invention.
Fig. 9 is a schematic view of a cell or tissue sealed with the cap being below a liquid surface of a coolant and a joint portion of the cap being above the liquid surface of the coolant, prior to a thawing step.
Fig. 10 is a schematic view of the body and the cap being separated from each other, and a deposition part being immersed in a thawing solution.

DESCRIPTION OF EMBODIMENTS

The device for cryopreservation of the present invention is suitably used for cell or tissue cryopreservation called "vitrification method". The term "cell" herein encompasses not only a single cell but also a biological cell population composed of multiple cells. The cell population composed of multiple cells may be a cell population composed of a single kind of cells or may be a cell population composed of multiple kinds of cells. The tissue may be composed of a single kind of cells or may be composed of multiple kinds of cells, or may contain a non-cellular substance like an extracellular matrix in addition to the cells. The device for cryopreservation of the present invention can be particularly suitably used in egg or embryo cryopreservation.
The device for cryopreservation of the present invention is used in a series of operations including a freezing step of freezing cells or tissues deposited using a cryogenic coolant, a refrigerating step of maintaining the frozen state, and a thawing step of thawing and defrosting the cells or tissues in a thawing solution and recovering them.
The device for cryopreservation of the present invention can also be referred to as a "cell or tissue preservation jig", a "cell or tissue cryopreservation jig" or a "cell or tissue preservation device".
Hereinafter, the device for cryopreservation of the present invention is described in detail.
The device for cryopreservation of the present invention includes a body including a strip-shaped tip, and a cap capable of covering the tip of the body. The body is in the shape of a rod and has the tip on one end.
The tip of the device for cryopreservation of the present invention has a strip shape. The strip-shaped tip is provided in a manner that the lengthwise direction of the tip is in parallel with the longitudinal direction of the body. The tip having a strip shape allows the deposition part to be easily covered and protected by the cap.
The cap of the device for cryopreservation of the present invention has an inner cavity capable of housing and protecting the deposition part. Preferably, the cap has an open end and a closed end and has a substantially square pillar shape. Preferably, the inner cavity of the cap has a substantially cylinder shape.
The cap of the device for cryopreservation of the present invention is preferably freely attachable to and detachable from the body. The wording "freely attachable to and detachable from" means that the cap can be easily fitted and fixed to the body and can be also easily removed from the body without any special tool. To enable this, the body preferably has a fitting structural part on a side of the tip, and the fitting structural part preferably has a tapered structure or a threaded structure. From the standpoint of rapid and simple attachment/detachment, the fitting structural part more preferably has a tapered structure. In the case where the fitting structural part of the body has a threaded structure, the cap preferably also has a threaded structure. In the case where the fitting structural part of the body has a tapered structure, the cap preferably also has a tapered structure from the standpoint of more secure fitting and fixing.

The cap of the device for cryopreservation of the present invention can be formed using, for example, a material resistant to coolants (e.g., liquid nitrogen) such as resins and metals. The cap may be made of one type or two or more types of materials. In particular, preferred are resins as they can easily form a tapered structure or a threaded structure through a process such as injection molding. Specific examples of the resin include polyester resins such as polyethylene terephthalate and polyethylene naphthalate, acrylic resin, epoxy resin, silicone resin, polycarbonate resin, diacetate resin, triacetate resin, polyacrylate resin, polyvinyl chloride resin, polysulfone resin, polyether sulfone resin, polyimide resin, polyamide resin, polyolefin resin, and cyclic polyolefin resin. When the cap has a total light transmittance of 80% or higher, advantageously, the deposition part inside the cap after fitting thereof can be easily checked.

The cap included in the device for cryopreservation of the present invention preferably has a fixing portion to be fixed to a fixture used prior to the thawing step. The fixing portion of the cap may have any irregular shape such as a recess or a projection according to the shape of the fixture.

The tip of the body included in the device for cryopreservation of the present invention includes at least a deposition part including a preservation solution absorber. The deposition part including a preservation solution absorber can effectively remove an excess preservation solution surrounding the cell or tissue owing to the preservation solution absorber, which improves operation efficiency during deposition and freezing of a cell or tissue. Examples of the preservation solution absorber include films made of wire mesh, paper, synthetic resin, or the like and having through holes. Another example of the preservation solution absorber may be a porous structure formed from a material having a refractive index of 1.45 or less. Owing to the porous structure, the preservation solution surrounding the cell or tissue can be removed. The porous structure also makes it possible to deposit and freeze a cell or tissue and thaw the cell or tissue after freezing with good visibility in an easy and reliable manner under transmission optical microscope observation.

Owing to the preservation solution absorber included in the deposition part, the device for cryopreservation of the present invention can make a preservation solution deposited on the deposition part flat. When the cell or tissue deposited is frozen and solidified using a cryogenic coolant, the preservation solution surrounding the cell or tissue is integrated with the part absorbed in the preservation solution absorber. As a result, the cell or tissue and the surrounding preservation solution frozen and solidified using a cryogenic coolant are surely held on the deposition part without falling off the deposition part.

The refractive index of the material of the porous structure described above can be measured using, for example, an Abbe's refractometer (Na light source; wavelength: 589 nm) in accordance with JIS K 0062:1992 and JIS K 7142:2014. Examples of the material having a refractive index of 1.45 or less to form the porous structure include plastic resin materials such as silicone resin and fluororesin (e.g., polytetrafluoroethylene resin, polyvinylidene difluoride resin, and polychlorotrifluoroethylene resin), metal oxide materials such as silicon dioxide, and inorganic materials such as sodium fluoride, magnesium fluoride, and calcium fluoride.

The pore size of the preservation solution absorber made of the porous structure is preferably 5.5 $\mu$m or less, more preferably 1.0 $\mu$m or less, still more preferably 0.75 $\mu$m or less. This can improve visibility of the cell or tissue under optical microscope observation. The thickness of the preservation solution absorber is preferably 10 to 500 $\mu$m, more preferably 25 to 150 $\mu$m. When the preservation solution absorber is a porous structure made of a plastic resin material, the pore size of the preservation solution absorber is the diameter of the largest pore measured by the bubble point test. When the preservation solution absorber is a porous structure made of a metal oxide or an inorganic material, the pore size is the average pore diameter determined by image observation of the surface and cross section of the porous structure.

The porosity of the preservation solution absorber is preferably 30% or more, more preferably 70% or more. The porosity is defined by a formula shown below. To determine the void volume V, a mercury porosimeter (name: Autopore II 9220, Micromeritics Instrument Corporation) is used to measure and process the cumulative pore volume (mL/g) of pores having a pore radius of 3 nm to 400 nm in the preservation solution absorber, and the cumulative pore volume (mL/g) is multiplied by the dry solids content (g/m$^2$) of the preservation solution absorber, whereby the void volume V can be determined as the value per unit area (m$^2$). The thickness T of the preservation solution absorber can be measured on a photograph of the cross section of the preservation solution absorber taken with an electron microscope.

$$P = (V/T) \times 100 \ (\%)$$

P: porosity (%)
V: void volume (ml/m$^2$)
T: thickness ($\mu$m)

The tip of the body included in the device for cryopreservation of the present invention may include a support in addition to the deposition part including a preservation solution absorber. Examples of the support include various resin films, metal plates, glass plates, and rubber plates. The support may be made of one type or two or more types of materials. Among these, resin films are suitably used from the standpoint of handleability. Specific examples of the resin film include resin films made of polyester resins such as polyethylene terephthalate and polyethylene naphthalate, acrylic resin, epoxy resin, silicone resin, polycarbonate resin, diacetate resin, triacetate resin, polyacrylate resin, polyvinyl chloride resin, polysulfone resin, polyether sulfone resin, polyimide resin, polyamide resin, polyolefin resin, and cyclic polyolefin resin.

In the case where the tip of the body included in the device for cryopreservation of the present invention includes a support, the support may also preferably be a metal plate because it has excellent thermal conductivity and enables rapid freezing. Specific examples of the metal plate include copper, copper alloy, aluminum, aluminum alloy, gold, gold alloy, silver, silver alloy, iron, and stainless steel.

Preferably, the various resin films, metal plates, glass plates, rubber plates, and the like described above each have a thickness of 10 $\mu$m to 10 mm. Depending on the purpose, it is possible to hydrophilize surfaces of these various resin films, metal plates, glass plates, rubber plates, and the like by an electrical method such as corona discharge treatment or a chemical method, and further to roughen these surfaces.

The tip of the body included in the device for cryopreservation of the present invention has a strip shape. In the plane of the sheet-shaped strip, the length in the minor axis direction perpendicular to the lengthwise direction of the strip which is set as the major axis direction (width of the strip, namely, the short side of the strip) is not shorter than 70% of the inner diameter of the cap. The inner diameter of the cap is the maximum diameter of the inner cavity, as viewed from the opening side of the cap. When the length in the minor axis direction of the tip is within the above range, upon insertion of the deposition part on which a cell or tissue is deposited with a preservation solution into the cap for housing of the deposition part, the preservation solution and the cell or tissue can be covered with the cap without risk of contact thereof with the end portion on the opening side of the cap to fall. In a case where the length in the minor axis direction of the tip is shorter than 70% of the inner diameter of the cap, the preservation solution and the cell or tissue may contact the end portion on the opening side of the cap to fall when the tip is bent during the above housing operation. The length in the minor axis direction of the strip-shaped tip is preferably not shorter than 70% and not longer than 95% of the inner diameter of the cap, because the working efficiency upon insertion is favorable. The sides (long sides) in the major axis direction of the strip-shaped tip preferably extend in parallel with each other (strip having a constant width). Still, the sides may not extend in parallel with each other as long as the center line of the strip extends linearly in the major axis direction. In such a case, the length in the minor axis direction of the tip is set as the maximum width of the strip. Moreover, the length in the minor axis direction of the tip is preferably shorter than the minimum diameter of the inner cavity of the cap.

The length in the major axis direction of the inner cavity of the cap included in the device for cryopreservation of the present invention is not longer than 200% of the length in the major axis direction of the tip of the body. The length in the major axis direction of the inner cavity of the cap refers to the length of the space of the inner cavity from the open end to the closed end of the cap in the direction along which the space of the inner cavity of the cap extends. When the length in the major axis direction of the inner cavity of the cap is within the above range, favorable working efficiency can be achieved in the operation of attaching the cap to the tip by inserting the deposition part on which a cell or tissue is deposited into the cap for covering the deposition part of the tip upon the freezing operation. In the thawing step, the body including the tip and the cap can be rapidly separated. A known case where the length in the major axis direction of the inner cavity of the cap is longer than 200% of the length in the major axis direction of the body member is, for example, a cryopreservation jig having a fitting structural part such as a tapered structure on a side closer to a handle, as described and illustrated in Patent Literature 11. With such a structure, it is difficult to rapidly separate the cap from the body in the thawing step. From the standpoint of covering and protection of the tip with the cap, the length in the major axis direction of the inner cavity of the cap included in the device for cryopreservation of the present invention is preferably not shorter than 105% of the length in the major axis direction of the tip.

The strip-shaped tip of the body included in the device for cryopreservation of the present invention has a length in the minor axis direction of not shorter than 70% of the inner diameter of the cap, and the inner cavity of the cap has a length in the major axis direction of not longer than 200% of the length in the major axis direction of the tip of the body. With this structure, the cell or tissue inside the inner cavity of the cap can be rapidly cooled when the strip-shaped tip having a deposition part, after being covered with the cap and fitted thereto to be fixed, is immersed in a coolant to be cooled and frozen in the freezing operation. For example, a conventionally known cryopreservation jig as disclosed and illustrated in Patent Literature 6 has a cap having an inner cavity which is large relative to the tip having a deposition part, which may unfavorably cause a failure in rapid cooling.

The body included in the device for cryopreservation of the present invention preferably has a handle from the standpoint of working efficiency. In that case, the handle preferably has a prism shape in order to improve grippability and handleability. Preferably, the handle is a part formed from a material resistant to coolants such as liquid nitrogen. Preferred examples of such a material include various metals such as aluminum, iron, copper, and stainless steel, ABS resin, acrylic resin, polypropylene resin, polyethylene resin, fluororesin, various engineering plastics, and glass.

In the case where the device for cryopreservation of the present invention has a body with a handle, the handle may have a marking on the front or the back for the purpose of facilitating distinguishing between the front and the back of the deposition part. For the same purpose, a characteristic structure (e.g., a recess or a projection) may be formed at a portion of the front of the handle.

The device for cryopreservation of the present invention has been described so far. Next, an exemplary freezing and thawing method that uses the device for cryopreservation of the present invention is described.

The freezing and thawing method that uses the device for cryopreservation of the present invention includes a freezing step, a refrigerating step, and a thawing step. In the freezing step, first, a cell or tissue is deposited with a preservation solution on the deposition part of the device for cryopreservation. Preferably, the operation is performed under transmission microscope observation (hereinafter, also simply described as "under microscope observation"). Here, since the deposition part of the device for cryopreservation includes a preservation solution absorber, advantageously, an excess preservation solution can be effectively removed, resulting in high handleability and less preservation solution surrounding the cell or tissue. This easily achieves a high freezing rate and a high thawing rate.

Next, the tip including the deposition part of the body is inserted into the cap so that the cap is fitted and fixed. The tip may be inserted into the cap under microscope observation. Since the tip included in the device for cryopreservation of the present invention has a length in the minor axis direction of not shorter than 70% of the inner diameter of the cap and a preservation solution droplet deposited on the deposition part has a flat shape due to the action of the preservation solution absorber, the tip including the deposition part can be covered with the cap without risk of falling of the cell or tissue due to a contact of the preservation solution and the cell or tissue with the end portion of the cap. The tip inserted into the cap is completely covered by the cap, and the cap is fitted and fixed to a fitting structural part of the body for complete sealing.

Then, the cap fitted and fixed to the body is immersed in a coolant such as liquid nitrogen, and the cell or tissue deposited on the deposition part is cooled and frozen. Here, the deposition part covered by the cap and completely sealed, and the cell or tissue on the deposition part are cooled without contact with the coolant. The strip-shaped tip of the body included in the device for cryopreservation of the present invention has a length in the minor axis direction of not shorter than 70% of the inner diameter of the cap and the inner cavity of the cap has a length in the major axis direction of not longer than 200% of the length in the major axis direction of the tip of the body. With this structure, favorable working efficiency can be achieved in insertion of the tip into the cap and fitting of the cap to the fitting structural part for fixture. Moreover, the cell or tissue deposited on the deposition part inside the inner cavity of the cap can be rapidly cooled.

Preferably, it takes not more than one minute from when the cell or tissue is deposited with the preservation solution on the deposition part and the deposition part is sealed to when the body sealed with the cap in the sealing operation is immersed in the coolant. More preferably, it takes not more than 30 seconds. In the case where the freezing step takes more than one minute, the cell or tissue may be strongly affected by the preservation solution containing a cryoprotectant, which is not favorable from the standpoint of viability. Since the inner cavity of the cap included in the device for cryopreservation of the present invention has a length in the major axis direction of not longer than 200% of the length in the major axis direction of the tip of the body, excellent working efficiency can be achieved even in the operation with such a time limit.

Then, the cooled cell or tissue is refrigerated in a cold storage container maintained at a cryogenic temperature by a coolant or the like, while the vitrification state of the cell or tissue is maintained. This process is a so-called refrigerating step.

In the case where the cap included in the device for cryopreservation of the present invention has a fixing portion, the device for cryopreservation can be held, prior to the thawing step, using a fixture capable of fixing the cap and the fixing portion favorably included in the cap. When the device for cryopreservation of the present invention fixed to the fixture using the fixing portion of the cap is immersed in the coolant together with the fixture, advantageously, the device for cryopreservation is easily temporarily held in a state in which the cell or tissue sealed with the cap is below a liquid surface of the coolant and a joint portion of the cap of the device for cryopreservation is above the liquid surface of the coolant.

Preferably, the fixture has a slit into which the cap of the device for cryopreservation is inserted to be fixed. Preferably, the slit has a slit fixing part for fixing the fixing portion of the cap. The fixture may have one slit or multiple slits.

Preferably, the fixture in the present invention is formed from a material resistant to coolants such as liquid nitrogen, as is the case for the cap described above. Preferred examples of such a material include various metals such as aluminum, iron, copper, and stainless steel, ABS resin, acrylic resin, polypropylene resin, polyethylene resin, fluororesin, various engineering plastics, and glass. Preferred is a metallic fixture as its heavy self-weight enables favorable fixing of the device for cryopreservation.

The device for cryopreservation of the present invention, prior to the thawing step, is preferably temporarily held in a state in which the deposition part is sealed with the cap and the position of the cell or tissue deposited on the deposition part is inside the coolant while a joint portion between the body and the cap is outside the coolant. Subsequently, preferably, the fitting and fixing between the cap and the body is loosened, and the cap is then separated from the body. After the separation, the deposition part is preferably immersed in a thawing solution (thawing step). These operations prior to the thawing step allow the deposition part of the body to be rapidly immersed in the thawing solution without contact with a coolant such as liquid nitrogen.

Preferably, it takes not more than five minutes from when the fitting and fixing between the body and the cap is loosened to when the deposition part is immersed in the thawing solution in the thawing step. More preferably, it takes not more than one minute. When the fitting and fixing is loosened and the sealed state is released for a long time, a gas such as nitrogen or oxygen may enter the cap, and then the gas may be cooled and liquefied. Disadvantageously, the liquefied gas which is attached to the deposition part and brought into the thawing solution may decrease the temperature of the thawing solution.

Since the inner cavity of the cap included in the device for cryopreservation of the present invention has a length in the major axis direction of not longer than 200% of the length in the major axis direction of the tip of the body, the working efficiency in separation of the cap from the body in the thawing step is favorable. In particular, a rapid thawing rate desired in a vitrification method can be easily achieved. The cell or tissue on the deposition part in the cryogenic state can be transferred into a thawing solution preferably within less than one second. The cell or tissue is more preferably transferred within 0.75 seconds.

The device for cryopreservation of the present invention is suitably used in the freezing and thawing method described above. Use of the device for cryopreservation of the present invention is preferred in the freezing step and the thawing step described above, because the air bubble attachment to the deposition part is reduced or prevented during recovery of the cell or tissue on the deposition part immersed in a thawing solution, which leads to excellent working efficiency in recovery in the thawing step.

The freezing and thawing method that uses the device for cryopreservation of the present invention has been described so far. Next, the device for cryopreservation of the present invention and the fixture favorably used in the freezing and thawing method that uses the device for cryopreservation of the present invention are described in further detail with reference to drawings.

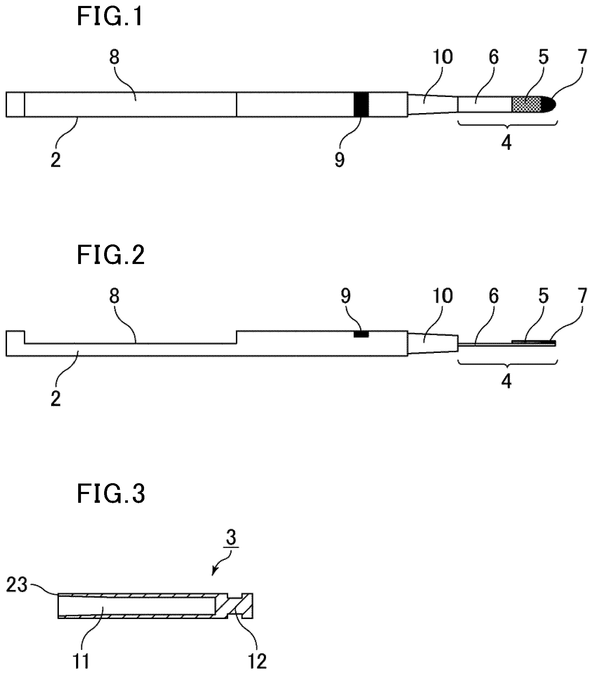

Fig. 1 is a schematic top view of an example of the body included in the device for cryopreservation of the present invention. A body 2 illustrated in Fig. 1 includes a handle 8, a fitting structural part 10, and a tip 4. The tip 4 includes a deposition part 5, a support 6, and a tip marking 7, and is attached to a fitting structural part 10. The handle 8 has a handle marking 9 for distinguishing a side on which a cell or tissue is to be deposited. The end of the tip 4, i.e., the tip marking 7 has a substantially hemicircular shape to enhance the working efficiency upon insertion into the cap. The fitting structural part 10 is a part for fixing the cap to the body 2. In Fig. 1, the fitting structural part 10 has a tapered structure in a truncated cone shape tapering from the handle 8 toward the tip 4.

Fig. 2 is a schematic side view of an example of the body included in the device for cryopreservation of the present invention. The handle 8 of the body 2 illustrated in Fig. 2 includes a recess at a portion thereof, which facilitates distinguishing between the front and the back of the tip 4, i.e., a side of the deposition part 5 on which a cell or tissue is to be deposited, when an operator grips the body 2. Similarly, a handle marking 9 is provided for the purpose of distinguishing between the front and the back of the tip 4. The tip 4 of the body 2 includes the deposition part 5 made of a preservation solution absorber and the tip marking 7 on the support 6. The deposition part 5 can be provided on the support 6 by bonding two short sides of the deposition part 5 to the support 6 with an adhesive layer interposed therebetween (the adhesive layer is not illustrated in Fig. 2.).

Fig. 3 is a side cross-sectional view of an example of the cap included in the device for cryopreservation of the present invention. Fig. 3 illustrates a structure of an inner cavity 11 of the cap 3 for covering the tip 4 of the body 2. The cap 3 has a structure in which only one side is open. A tapered structure for fitting the cap 3 to the fitting structural part 10 of the body 2 is formed in the vicinity of an opening 23. The cap 3 includes a fixing portion 12 for fixing the cap 3 to the fixture prior to the thawing step. The fixing portion 12 has a shape of a recess or projection in accordance with the shape of a fixture 16 described later.

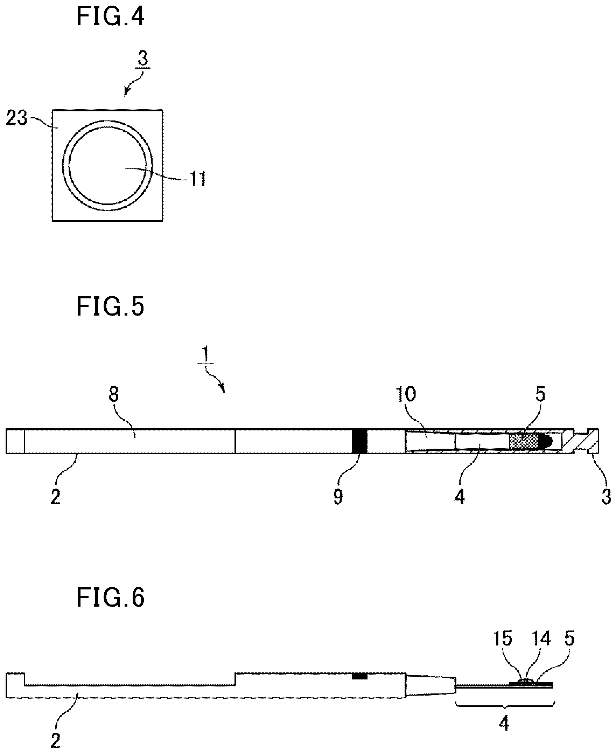

Fig. 4 is a side view of an example of the cap of the device for cryopreservation of the present invention, as viewed from an opening side. As illustrated in Fig. 4, the cap 3 has an external shape in the form of a quadrangular prism and has the inner cavity 11 in the form of a cylinder shape.

Fig. 5 is a schematic view of an example of the body 2 and the cap 3 being fitted together and fixed. In Fig. 5 and some of the subsequent figures, the internal structure (cross-sectional structure) of the cap 3 is illustrated to show the state of the fitting structural part 10 and the tip 4 covered by the cap 3. In the device for cryopreservation 1 illustrated in Fig. 5, the cap 3 is completely fitted and fixed to the body 2 by the fitting structural part 10 of the body 2 and the tip 4 is covered and sealed with the cap 3 and is thus completely protected from the outside environment. In the case where the material used in the cap does not have sufficient transparency, the handle marking 9 is preferably provided because distinguishing between the front and the back of the tip 4 is difficult.

Fig. 6 is a schematic view of a cell and a preservation solution being deposited on the device for cryopreservation. In the freezing and thawing method that uses the device for cryopreservation 1 of the present invention, an operator drops a cell 14 and a preservation solution 15 to the deposition part 5 attached to the tip 4 of the body 2 to deposit the cell 14 on the deposition part 5. A droplet of the deposited preservation solution 15 changes its shape into a flat shape on the deposition part 5 along with automatic removal of an excess preservation solution by the deposition part 5 made of a preservation solution absorber.

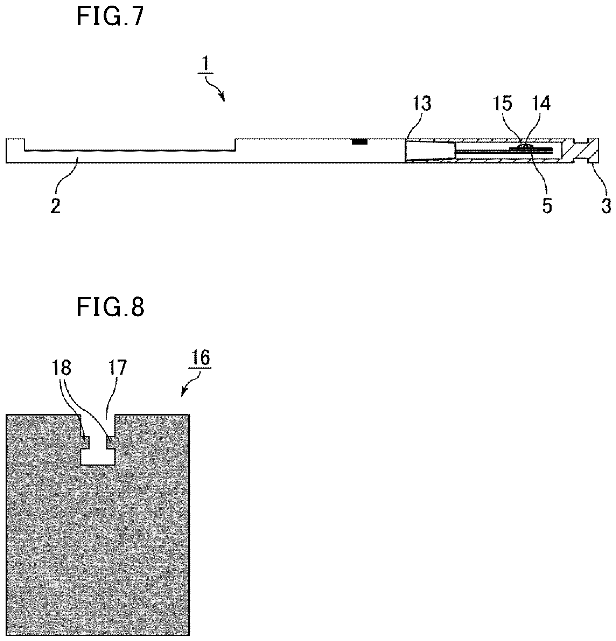

Fig. 7 is a schematic view of the cap being fitted and fixed after the cell and the preservation solution are deposited on the device for cryopreservation of the present invention. The cell 14 is dropped with the preservation solution 15 to the deposition part 5 of the body 2 and is deposited on the deposition part 5. Then, the cap 3 is fitted and fixed to the body 2. Thus, the cell 14 deposited on the deposition part 5 is completely covered and sealed with the cap 3 and completely protected from the outside environment. The boundary between the cap 3 and the body 2 is a joint portion 13.

Fig. 8 is a schematic side cross-sectional view of an example of a fixture used in the freezing and thawing method that uses the device for cryopreservation of the present invention. In Fig. 8, a fixture 16 has a slit 17 into which the cap 3 is inserted, and the slit 17 has a slit fixing part 18 for fixing the fixing portion 12 of the cap 3.

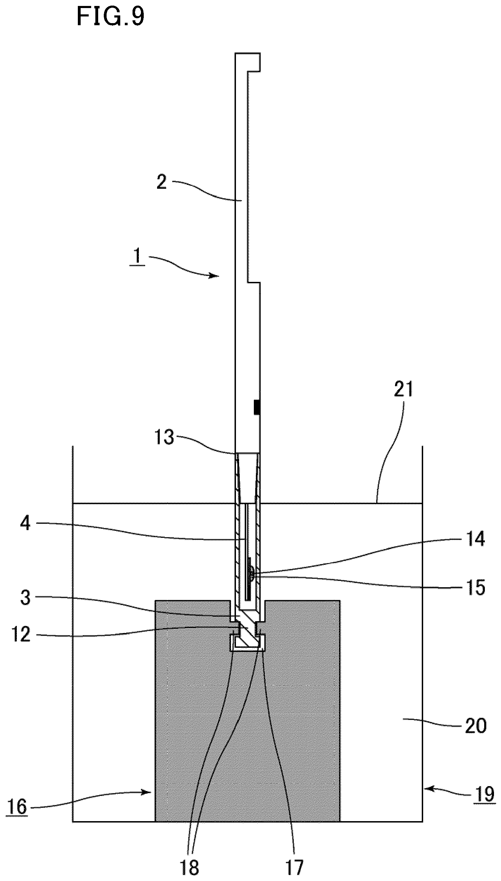

Fig. 9 is a schematic view of the cell or tissue sealed with the cap being below a liquid surface of a coolant and a joint portion of the cap being above the liquid surface of the coolant, prior to the thawing step. In Fig. 9, the fixing portion 12 of the cap 3 included in the device for cryopreservation 1 is inserted into the slit fixing part 18 formed at a portion of the slit 17 of the fixture 16 to be held and fixed. At this time, the joint portion 13 between the cap 3 and the body 2 is above a liquid surface 21 of a coolant 20 filling a freezing container 19. Thus, even when the fitting and fixing between the body 2 and the cap 3 included in the device for cryopreservation 1 is loosened and the fitting is released, the surrounding coolant is prevented from directly entering the cap 3. In other words, the coolant does not contact any of the tip 4, the cell 14, and the preservation solution 15, while the cooling state of the cell or tissue is maintained to sufficiently cool the cell 14. This enables rapid thawing in the subsequent thawing step by transferring the cell or tissue in the cryogenic state into a thawing solution. In other words, the cell or tissue can be rapidly thawed without a decrease in the thawing rate.

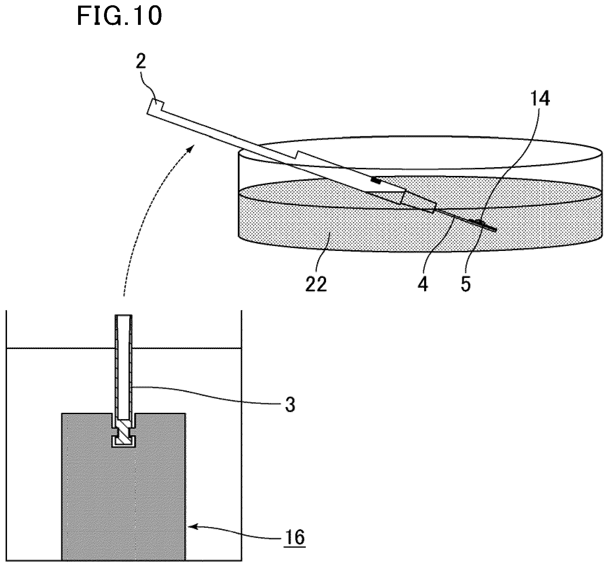

Fig. 10 is a schematic view of the body and the cap being separated from each other, and the deposition part being immersed in a thawing solution. In Fig. 10, the cap 3 is fixed to the fixture 16, so that the body 2 including the tip 4 and the deposition part 5 attached to the tip 4 can be rapidly separated from the cap 3 by simply pulling out the body 2. After separation, the deposition part 5 is immersed in a thawing solution 22, and the cell 14 is thawed and recovered in the thawing solution 22.

When cryopreserving and thawing the cell or tissue by the freezing and thawing method that uses the device for cryopreservation of the present invention, the preservation solution may be one commonly used for freezing cells, such as eggs and embryos. For example, the preservation solution may be the above-described preservation solution prepared by adding a cryoprotectant (e.g., glycerol, ethylene glycol, or dimethyl sulfoxide (DMSO)) to a physiological solution such as a phosphate buffered saline, or a preservation solution containing a large amount (at least 10 mass% or more, more preferably 20 mass% or more relative to the total mass of the preservation solution) of a cryoprotectant. The thawing solution may be one that is commonly used to thaw cells such as eggs and embryos. For example, a thawing solution prepared by adding 1 M sucrose for osmoregulation to the above-described physiological solution such as phosphate buffered saline may be used.

Examples of the cell that can be used in the freezing and thawing method that uses the device for cryopreservation of the present invention include reproductive cells such as eggs, embryos, and sperms from mammals (for example, human, bovine, swine, equine, leporine, rat, and mouse); and pluripotent stem cells such as induced pluripotent stem cells (iPS cells) and embryonic stem cells (ES cells). Examples also include culture cells such as primary culture cells, subculture cells, and cell lines. In one or more embodiments, examples of the cell include adhesive cells such as fibroblasts, cancer-derived cells (e.g., pancreatic cancer cells and hepatoma cells), epithelial cells, vascular endothelial cells, lymphatic endothelial cells, neuronal cells, chondrocytes, tissue stem cells, and immune cells. Examples of the tissue that can be cryopreserved and thawed include tissues formed of homologous cells and tissues formed of heterologous cells, such as tissues of ovary, skin, corneal epithelium, periodontal ligament, and myocardium. The present invention is particularly suitable for cryopreservation of cells having a sheet-shaped structure (e.g., cell sheet, skin tissue). The device for cryopreservation of the present invention is suitably used not only for cryopreservation of tissues directly harvested from living bodies but also for cryopreservation of artificial tissues such as skins cultured in vitro, cell sheets formed in vitro, or organizational models having a three-dimensional structure suggested in JP 2012-205516 A. The device for cryopreservation of the present invention is suitably used as the device for cryopreservation of a cell or tissue described above.

EXAMPLES

The present invention is specifically described in more detail below with reference to examples, but the present invention is not limited to the examples below.

(Example 1)

A device for cryopreservation of Example 1 having a configuration as illustrated in Fig. 1 and Fig. 3 was produced. A polyethylene terephthalate resin film (total light transmittance: 89%, haze: 2.3%) was used as a support. To the support was attached a polytetrafluoroethylene porous body (pore size: 0.2 $\mu$m, porosity: 71%, thickness: 35 $\mu$m) available from Advantec Toyo Kaisha, Ltd. as a preservation solution absorber, using a hot-melt urethane resin Purmelt® QR 170-7141P available from Henkel Japan Ltd. as an adhesive layer in a manner that only two ends on the minor axis side were bonded to the support to be fixed. Thus, a tip including a deposition part was produced. A handle and fitting structural part of the body and a cap were produced using ABS resin. The tip was joined with the handle and the fitting structural part. Thus, the device for cryopreservation of Example 1 was produced. The tip of the body included in the device for cryopreservation of Example 1 had a length in the minor axis direction of 1.75 mm and a length in the major axis direction of 20 mm. The cap included in the device for cryopreservation of Example 1 had an inner diameter of 2.1 mm and a length of its inner cavity in the major axis direction of 35 mm.

(Example 2)

A device for cryopreservation of Example 2 was produced as in Example 1, except that the length in the minor axis direction of the tip of the body was changed from 1.75 mm to 1.5 mm.

(Example 3)

A device for cryopreservation of Example 3 was produced as in Example 2, except that the length in the major axis direction of the inner cavity of the cap was changed from 35 mm to 40 mm.

(Comparative Example 1)

A device for cryopreservation of Comparative Example 1 was produced as in Example 2, except that no preservation solution absorber was provided and the support was used as a deposition part as it was.

(Comparative Example 2)

A device for cryopreservation of Comparative Example 2 was produced as in Example 1, except that the length of the tip of the body in the minor axis direction was changed from 1.75 mm to 0.7 mm.

(Comparative Example 3)

A cap was produced in a manner that the inner cavity of the cap had a length in the major axis direction of 80 mm. A device for cryopreservation of Comparative Example 3 was produced as in Example 1, except that the shape of the fitting structural part was enlarged toward the handle side while the length of the body and the total length of the device for cryopreservation when the cap was fitted and fixed to the body were the same as those of the device for cryopreservation of Example 1.

<Evaluation on working efficiency in insertion into cap in freezing step>

On the deposition part of each of the devices for cryopreservation of Examples 1 to 3 and Comparative Examples 1 to 3 was dropped a glass bead (diameter: 100 $\mu$m) as a false cell with a preservation solution in an amount of 0.1 $\mu$L under a transmission microscope. The preservation solution had a composition containing DMSO (15 vol%), ethylene glycol (15 vol%), and sucrose (17 mass%) in Medium 199 available from Sigma-Aldrich. Then, the tip including the deposition part was inserted into the cap under a transmission microscope. Here, whether or not the preservation solution on the deposition part contacts the end face on the opening side of the cap was checked. This operation was performed five times, and the "Evaluation on working efficiency in insertion into cap in freezing step" was made based on the following criteria. Table 1 shows the results.

Good: The preservation solution did not contact the end face on the opening side of the cap even once out of five operations.

Poor: The preservation solution contacted the end face on the opening side of the cap at least once out of five operations.

<Evaluation on working efficiency in immersion into thawing solution in thawing step>

In the same manner as described above, on the deposition part of each of the devices for cryopreservation of a cell or tissue of Examples 1 to 3 and Comparative Examples 1 to 3 was dropped a false cell with a preservation solution and the cap was completely fitted and fixed to the body. Then, the jig was immersed in liquid nitrogen to be frozen. After the refrigerating step, the body was fixed as illustrated in Fig. 9 to a fixture in the form illustrated in Fig. 8 prior to the thawing step. Then, fitting and fixing of the cap was slightly loosened while the false cell was maintained to be positioned below the liquid surface of liquid nitrogen but not in contact with liquid nitrogen. Next, as illustrated in Fig. 10, the body was pulled out while the cap was fixed to the fixture to separate the body from the cap and the tip of the body was immersed in a thawing solution. The time needed for this operation was measured to make an "evaluation on working efficiency in immersion into thawing solution in thawing step" based on the following criteria. The thawing solution had a composition containing sucrose (34 mass%) in Medium 199. Table 1 shows the results.

Excellent: The time needed for the operation was not longer than 0.75 seconds.
Good: The time needed for the operation was longer than 0.75 seconds and not longer than one second.
Poor: The time needed for the operation was longer than one second.

[Table 1]

| | Evaluation on working efficiency in insertion into cap member in freezing step | Evaluation on working efficiency in immersion into thawing solution in thawing step |
|---|---|---|
| Example 1 | Good | Excellent |
| Example 2 | Good | Excellent |
| Example 3 | Good | Excellent |
| Comparative Example 1 | Poor | Excellent |
| Comparative Example 2 | Poor | Excellent |
| Comparative Example 3 | Good | Poor |

<Evaluation on working efficiency during recovery in thawing step>

The tip including a deposition part of each of the devices for cryopreservation of Examples 1 to 3 and Comparative Examples 1 to 3 was immersed in the thawing solution in the same manner as in "Evaluation on working efficiency in immersion into thawing solution in thawing step" described above. Then, the deposition part and the false cell on the deposition part in the thawing solution were observed under a microscope. Air bubble attachment to the deposition part observed in the thawing solution was checked. Air bubble attachment to the deposition part was not observed in any of the devices for cryopreservation of Examples 1 to 3 and Comparative Examples 1 to 3, which indicates that all the jigs have high working efficiency in recovery in the thawing step.

The above results show that the freeze thawing jig of the present invention exhibits excellent working efficiency in both the freezing step and the thawing step. In addition, the cell or tissue can be thawed in a very short time, which is expected to lead to high viability.

INDUSTRIAL APPLICABILITY

The present invention can be applied to cryopreservation and thawing of cells or tissues, such as cells or tissues for embryo transfer and artificial insemination of domestic animals (e.g., cattle) and other animals, and for human artificial insemination; iPS cells; ES cells; commonly used culture cells; cells or tissues, including embryos and eggs, harvested from living bodies for the purpose of examination or implantation; and cells or tissues cultured in vitro.

REFERENCE SIGNS LIST

    1 device for cryopreservation of cell or tissue
    2 body
    3 cap
    4 tip
    5 deposition part
    6 support
    7 tip marking
    8 handle
    9 handle marking
    10 fitting structural part
    11 inner cavity of cap
    12 fixing portion
    13 joint portion
    14 cell
    15 preservation solution
    16 fixture
    17 slit
    18 slit fixing part
    19 freezing container
    20 coolant
    21 liquid surface
    22 thawing solution
    23 opening


**Claims**

1.  A device for cryopreservation of a cell or tissue comprising at least:

    a body comprising a strip-shaped tip; and
    a cap covering the tip of the body,
    the tip comprising at least a deposition part comprising a preservation solution absorber,
    the tip having a length in a minor axis direction of not shorter than 70% of an inner diameter of the cap,
    the cap comprising an inner cavity having a length in a major axis direction of not longer than 200% of a length in a major axis direction of the tip.

FIG.1

FIG.2

FIG.3

FIG.4

FIG.5

FIG.6

FIG.7

FIG.8

# FIG.9

FIG.10

## INTERNATIONAL SEARCH REPORT

| International application No. |
| --- |
| PCT/JP2020/030049 |

A.   CLASSIFICATION OF SUBJECT MATTER
Int.Cl. A01N1/02(2006.01)i, C12M1/00(2006.01)i, C12N5/071(2010.01)i
FI: C12M1/00A, C12N5/071, A01N1/02

According to International Patent Classification (IPC) or to both national classification and IPC

B.   FIELDS SEARCHED

Minimum documentation searched (classification system followed by classification symbols)
Int.Cl. A01N1/02, C12M1/00, C12N5/071

Documentation searched other than minimum documentation to the extent that such documents are included in the fields searched
Published examined utility model applications of Japan       1922-1996
Published unexamined utility model applications of Japan     1971-2020
Registered utility model specifications of Japan             1996-2020
Published registered utility model applications of Japan     1994-2020

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
| --- | --- | --- |
| X | WO 2016/031916 A1 (KITAZATO BIOPHARMA CO., LTD.) 03.03.2016 (2016-03-03), claims 1, 10, fig. 6, 12 | 1 |
| Y | JP 2012-140422 A (NIPRO CORPORATION) 26.07.2012 (2012-07-26), claim 1, paragraph [0024], fig. 1-4 | 1 |
| Y | JP 3202440 U (MITSUBISHI PAPER MILLS LIMITED) 04.02.2016 (2016-02-04), paragraphs [0011], [0025] | 1 |

☐   Further documents are listed in the continuation of Box C.       ☒   See patent family annex.

| | |
| --- | --- |
| *       Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    09.09.2020 | Date of mailing of the international search report<br>    24.09.2020 |
| --- | --- |
| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.

PCT/JP2020/030049

```
WO 2016/031916 A1  03.03.2016   US 2017/0164606 A1
                                claims 1, 10, fig. 6, 12
                                EP 3196290 A1

JP 2012-140422 A   26.07.2012   (Family: none)

JP 3202440 U       04.02.2016   (Family: none)
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3044323 B
- JP 2008005846 A
- JP 2017104061 A
- JP 2000189155 A
- JP 5798633 B
- JP 2002315573 A
- JP 2006271395 A
- JP 2014183757 A
- JP 2015142523 A
- WO 2015064380 A
- WO 2019004300 A
- JP 2012205516 A

**Non-patent literature cited in the description**

- **STEPONKUS et al.** *Nature,* 1990, vol. 345, 170-172
- ''Seisaibo no toketsu ni yoru shogai to hogo no kiko'' (Mechanism of damage to living cells by freezing and protection) written by Hiroshi Souzu, Kagaku to Seibutsu. Chemistry and Biology. The Japan Society for Bioscience, Biotechnology, and Agrochemistry, 1980, vol. 18, 78-87